# EUROPEAN PATENT APPLICATION

(11) **EP 3 858 279 A1**
(43) Date of publication of application: **04.08.2021**
(21) Application number: 20154362.6
(22) Date of filing: 29.01.2020
(51) Int. Cl.: A61B 34/10, A61F 2/38, A61F 2/30, A61F 2/40

(54) **DETERMINING A SIZE OF AN IMPLANT**

(71) Applicant: Ottokar-Kernsto Leibnitz, 6534 AT Nijmegen (NL)
(72) Inventor: van Tienen, Tony, 6534 AT Nijmegen (NL); Bitter, Thom, 6525 GA Nijmegen (NL)
(74) Representative: Nederlandsch Octrooibureau

(57) **Abstract**

A system for sizing an implant on a bony surface in a joint, comprising a storage means (1509) for storing a dataset comprising a three-dimensional shape model of an object. A fitting unit (1506) is provided for fitting a reference model to the shape model of the object, and defining a coordinate system relative to the fitted reference model. An extracting unit (1507) is provided for extracting shape related features from the shape model, in dependence on the coordinate system. A sizing unit (1508) is provided for determining a size of an implant, based on the extracted shape related features. The sizing unit (1508) is configured for comparing the extracted shape related features to corresponding features associated with a plurality of differently sized implants and selecting a particularly sized implant based on a result of the comparing.

## Description

### FIELD OF THE INVENTION

The invention relates to determining a size of an implant.

### BACKGROUND OF THE INVENTION

Implants, such as knee, hip, and shoulder implants, are important for patient treatment. For example, implants can help to reinforce certain portions of the skeleton. In particular joints may degenerate over time. Not only degeneration, but also traumas can make it necessary to place an implant. However, since each individual patient has a different shape and size, an optimal size for the implant has to be determined for each patient individually.

For example, the normal meniscus in the knee contains two crescent discs that consist of cartilage-like tissue. The menisci are positioned between the thighbone (femur) and shinbone (tibia). On cross-section, perpendicular to the surface, they are wedge-shaped. The menisci play a role in even distribution and transmission of the body load over the joint and have a shock-absorbing (cushioning) function. Stabilization and lubrication of the knee joint are other functions of the meniscus. Each knee has two menisci, a medial (inner) meniscus and a lateral (outside) meniscus. The medial and lateral menisci are different. The lateral meniscus covers a larger area of the tibia plateau, is wider and more important for weight transfer (approximately 70%). The medial meniscus provides approximately 50% weight transfer, is c-shaped, located towards the inside of the knee and is more rigidly fixed and more important for stability. This makes the medial meniscus less mobile than the lateral meniscus. This disparity in motion explains why medial meniscus injuries occur three times more frequently than lateral.

Meniscus tears are among the most common knee injuries. These can be acute, when the tear is due to trauma (forced movement or rotation) or chronic (degenerative), which usually occur in older patients when the meniscus has gradually weakened and has become degenerative, losing its mechanical properties and shock absorbing function.

When a tear in the meniscus is diagnosed, the first question to be asked is whether this tear can be sutured. The meniscus lesion type, size and location determine whether the surgeon can repair the tear by suturing. If the meniscus cannot be sutured, the torn parts of the meniscus are often removed by meniscectomy (partial or total removal of the meniscus). Although a meniscectomy in general reduces pain and restores knee function immediately after surgery, the underlying problem is the deficient meniscus tissue that increases the local pressure on the cartilage of the tibia and femur, which leads to cartilage degeneration and increased risk to knee osteoarthritis and future total knee replacement.

Knee osteoarthritis is a chronic joint disease that results from breakdown and wear of joint cartilage and ultimately the underlying bone. This decreases the natural cushioning in the knee joint, resulting in chronic joint pain, stiffness and swelling of the knee. These disease symptoms seriously affect the ability to move and people's daily life activities overall.

Currently the treatment options for knee pain after meniscectomy are limited to replacement with allograft (donor meniscus) tissue, bone realignment surgery (High Tibial Osteotomy) and total knee replacement surgery. Allografts have shown to successfully reduce pain and improve knee function in patients with meniscectomy. However, their availability is limited, and meniscal allograft transplantation is generally restricted to patients younger than 50 years. Total knee replacement surgeries, where a full metal knee joint replaces the native knee joint, are only possible for patients with severe articular cartilage damage. Due to its limited durability total knee joint replacement are more suitable for older patients as younger patients will require revision surgery later on, which is a more complex procedure with a high rate of complications. A meniscus implant that is able to preserve the patient's knee and delay total knee replacement as long as possible may serve as an alternative to meniscal allografts.

WO 2015/057056 A1 discloses a joint prosthesis body made of a first biocompatible non-resorbable material and an anchoring element made of a second biocompatible non-resorbable material. The anchoring element comprises a plug and a cable with a disc at its distal end. To secure the prosthesis in place, the cable is passed through the bore in the prosthesis and the plug secured within a bone tunnel.

WO 2015/135907 A1 discloses a meniscus prosthesis comprising an arc-shaped meniscus prosthesis body having a main portion comprising a reinforcing part and two end portions comprising fixation parts, wherein the main portion comprises a part made of a first biocompatible, non-resorbable material extending between the two end portions, wherein the reinforcing part and the fixation parts are made of a second biocompatible, non-resorbable material and wherein the reinforcing part extends between the fixation parts and wherein the fixation parts have a through hole.

WO 2019/125167 A1 discloses a prosthesis assembly for orthopedic implantation comprising a prosthesis body and an attachment portion coupled to the prosthesis body for attaching the prosthesis assembly to a bone by way of a bone anchor or other fixating means. The attachment portion comprises an opening having a receiving portion with a first cross-sectional transverse width and a retaining portion extending from the receiving portion, the retaining portion having a second cross-sectional transverse width, which is less than the first cross-sectional transverse width.

A meniscus prosthesis may be an anatomically shaped, non-biodegradable polymer meniscus implant designed to mimic the function of the natural meniscus and functions as a shock absorber by adequate load distribution within the knee joint. The meniscus prosthesis may be implanted during an arthroscopic procedure.

The meniscus prosthesis may be made from a medical grade polymer with excellent mechanical and friction properties, thereby evenly distributing loads over the joint cartilage and having a shock-absorbing (cushioning) function. This polymer polycarbonate urethane (PCU) has an extensive history of safe use as orthopedic, spine and cardiac implants. After implantation and rehabilitation, the meniscus prosthesis is intended to provide relief of the chronic pain and full functionality of the knee. The treatment of meniscus defects with a meniscus implant can provide an additional treatment option next to meniscus tear surgical repair and the donor meniscus and delays the need for a total knee replacement for patients with persistent knee pain following medial meniscus surgery.

During surgery, special measurement instruments may be used directly on the body of the patient to find the optimal size and location of the implant. These measurement instruments may provide an extra reference for the surgeon during the surgery; it does not exclude the use of other surgical instruments for placing an implant. With the sizing instruments the definite size of the implant may be determined, but should preferably not deviate too much from the pre-planned size and position.

### SUMMARY OF THE INVENTION

It is an object of the invention to provide a proper size of implant for a specific individual. To better address this concern, a system for sizing an implant is provided, comprising
a storage means configured to receive and store a dataset comprising a three-dimensional shape model of an object;
a fitting unit configured to fit a reference model to the shape model of the object, and define a coordinate system relative to the fitted reference model;
an extracting unit configured to extract shape related features from the shape model, based on the coordinate system;
a sizing unit configured to determine a size of an implant, based on the extracted shape related features.

By analysing a three-dimensional shape model in this way, it becomes possible to predict an appropriate size of the implant with greater accuracy. Moreover, the size may be obtained in a way that is relatively reproducible, easy, and efficient. The size of implant having been found out in advance, the surgery may be more efficient, since no measurements need to be taken during surgery.

In certain joint prostheses, sizing of the implant is an important step in the surgical procedure because the implant size determines the ratio between the loads transferred through the implant and the recipient's cartilage. A wrong size of implant can result in too much of the load being carried by either the cartilage or the implant, both of which are undesirable. When too much load is transferred through the cartilage, the cartilage could get damaged. When too much load is transferred through the implant, the implant could deform more than desired, which possibly could lead to early failure. From the same perspective, also the location of the implant fixation points is important for an optimal load distribution.

For example, the implant is an implant to be placed on a bony surface in a joint.

The sizing unit may be configured to compare the extracted shape related features to corresponding features associated with a plurality of differently sized implants; and select a particularly sized implant based on a result of the comparison. This allows to select one of a plurality of candidate implant sizes by comparing the features of the shape model to the features associated with the candidate implants.

The implant may be configured to be fitted to the object. In case the implant is fitted or fixed to the object, it is important that the shape related features of the implant match those of the object to which the implant is fitted.

The object may comprise a bone. As an example of bone, the object may comprise at least part of a tibia. The bone, such as tibia, can be a suitable object to fix an implant to. Also, since the bone is rigid, it is important that the shape of the bone matches the shape of the implant. The system helps to determine the best size of the implant.

The implant may be a meniscus prosthesis. This type of prosthesis has to be able to carry a relatively high load. When the shape of the prosthesis is well adapted to the bone, the weights on the implant and/or on the bones of the patient may be reduced or more balanced.

The extracting unit may be configured to identify a region relative to the reference model, using the coordinate system, and searching for the features of the shape model in the identified region. This allows to detect the features that are relevant for sizing the implant more efficiently.

The extracting unit may be configured to extract information indicative of a curvature of an outside rim of the object. This rim may be relatively important relative to the implant, because it determines for a large portion the outer boundary of the implant as well.

The object may comprise at least part of a tibia. Further, the extracting unit may be configured to extract information indicative of a medial boundary of a proximal extremity of the tibia. This was found to be particularly effective for determining the size of a medial meniscus prosthesis.

Alternatively, the extracting unit may be configured to extract information indicative of a lateral boundary of a proximal extremity of the tibia. This was found to be particularly effective for determining the size of a lateral meniscus prosthesis.

The extracting unit may be configured to select a subset of the dataset corresponding to at least a portion of the shape model within a predetermined region, which region may be defined with respect to the coordinate system, where the implant is to be placed; fit a plane to the shape model based on the subset of the dataset; and determine intersection points of the shape model with the fitted plane. The sizing unit may be configured to select the size of the implant based on the determined intersection points. This provides an efficient manner to determine and use an estimation of the curvature of the outside rim of the object.

The sizing unit may be configured to compare the information indicative of the curvature of the outside rim of the object or the determined intersection points to a corresponding curve associated with the implant. The more similar the detected points are to the curve associated with the implant, the better that implant is suited for the patient.

The extracting unit may be configured to determine a surface of the shape model in a specific region relative to the coordinate system, and detect a dimple in the surface. The sizing unit may be configured to determine the size of the implant based on the detected dimple. Features of a dimple in the object may be related to certain features of an implant size.

The sizing unit may be configured for determining the size of the implant based on a location of the detected dimple. Such dimples may be useful recognition points of certain anatomical features of the patient, which may be mapped to certain positions on the implant to determine a suitable size of the implant.

The specific region may comprise a medial plateau of an upper extremity of a tibia. The region in which the dimples are detected may be the medial plateau of the upper extremity of a tibia, where a meniscus prosthesis may be put. The plateau was found to have dimples e.g. at the position of the anterior and posterior horns of the meniscus, in particular at the posterior horn. These horns correspond in turn to horns of the meniscus prosthesis. At these horns, in certain cases fixation elements may be provided, which may be used to fix the meniscus prosthesis at or near these dimples. Therefore, the location of the dimples may be important to match for successful implantation.

The specific region may correspond to a region within a predetermined distance from the fitted plane. In certain implementations, the plane is an estimation of a certain surface area of the shape model, for example the medial plateau of the tibia. To find the dimples in that surface, the portion of the surface close to the fitted plane may be analyzed.

The sizing unit may be configured to associate the location of the detected dimple with a location of an attachment element of the implant for attaching the implant to the object. This provides an important reference point for sizing the implant.

The system may further comprise a localizing unit configured to determine an implant position, where the implant can be placed, relative to the three-dimensional shape model, based on the extracted shape related features. This helps to allow a surgeon to be informed of the optimal implant location for the implant.

According to another aspect of the invention, a method is provided for determining a size of an implant. The method comprises:
providing a dataset comprising a three-dimensional shape model of an object;
fitting a reference model to the shape model of the object, and defining a coordinate system relative to the fitted reference model;
extracting shape related features from the shape model, in dependence on the coordinate system; and
determining a size of the implant, based on the extracted shape related features.

The method may further comprise providing an implant having the determined size.

According to another aspect of the invention, a computer program product is provided. The computer program product comprises instructions for causing a computer to perform the method set forth.

The person skilled in the art will understand that the features described above may be combined in any way deemed useful. Moreover, modifications and variations described in respect of the system may likewise be applied to the method and to the computer program product, and modifications and variations described in respect of the method may likewise be applied to the system and to the computer program product.

### BRIEF DESCRIPTION OF THE DRAWINGS

In the following, aspects of the invention will be elucidated by means of examples, with reference to the drawings. The drawings are diagrammatic and may not be drawn to scale. Throughout the drawings, similar items may be marked with the same reference numbers.
Fig. 1 shows a frontal perspective view of anatomical structures within a human knee.
Fig. 2 shows a top-down perspective view of anatomical structures within the human knee.
Fig. 3 shows a top-down perspective view of anatomical structures within a human knee, including a meniscus prosthesis.
Fig. 4 shows a shape model of a tibia and a fitting plane.
Fig. 5 shows intersection points of the tibia surface and the fitting plane
Fig. 6 shows a curve representing the rim of the tibia plateau.
Fig. 7 shows a plurality of different curves associated with different implant sizes.
Fig. 8 illustrates how to select points on the surface that correspond to horn attachment points.
Fig. 9 further illustrates how to select points on the surface that correspond to horn attachment points.
Fig. 10 shows measurements of the distance of the posterior horn-associated dimple to the detected curve.
Fig. 11 shows a tibia with a proper location and size of an implant.
Fig. 12 shows a tibia with a suboptimal implant size
Fig. 13 shows a tibia with an optimal implant size.
Fig. 14 shows a tibia with another suboptimal implant size.
Fig. 15 shows a block diagram of a system for determining a size of an implant.
Fig. 16 shows a flowchart of a method of determining a size of an implant.

### DETAILED DESCRIPTION OF EMBODIMENTS

Certain exemplary embodiments will be described in greater detail, with reference to the accompanying drawings.

The matters disclosed in the description, such as detailed construction and elements, are provided to assist in a comprehensive understanding of the exemplary embodiments. Accordingly, it is apparent that the exemplary embodiments can be carried out without those specifically defined matters. also, well-known operations or structures are not described in detail, since they would obscure the description with unnecessary detail.

Although the following description is focused on meniscus prosthesis sizing, it will be appreciated that the techniques are not limited thereto. For example, other

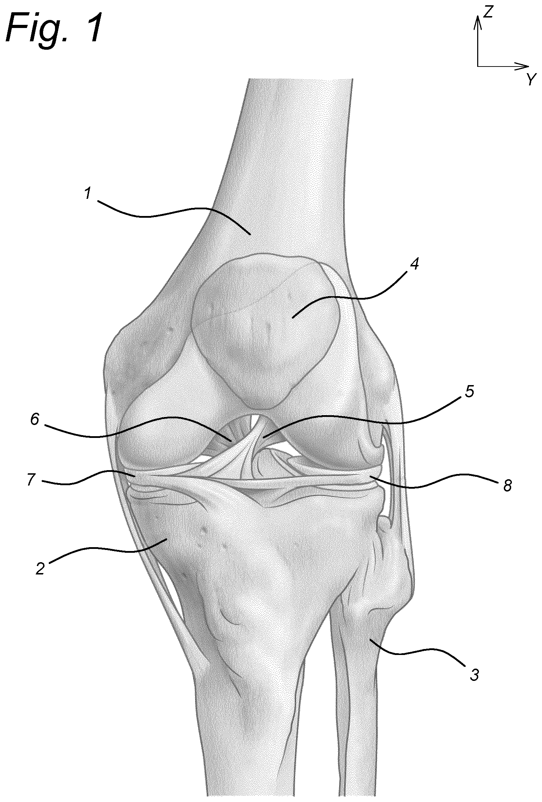

Fig. 1 shows a frontal perspective view of known anatomical structures within a human knee. The figure illustrates the femur 1, tibia 2, fibula 3, patella 4, anterior cruciate ligament 5, posterior cruciate ligament 6, medial meniscus 7, and lateral meniscus 8.

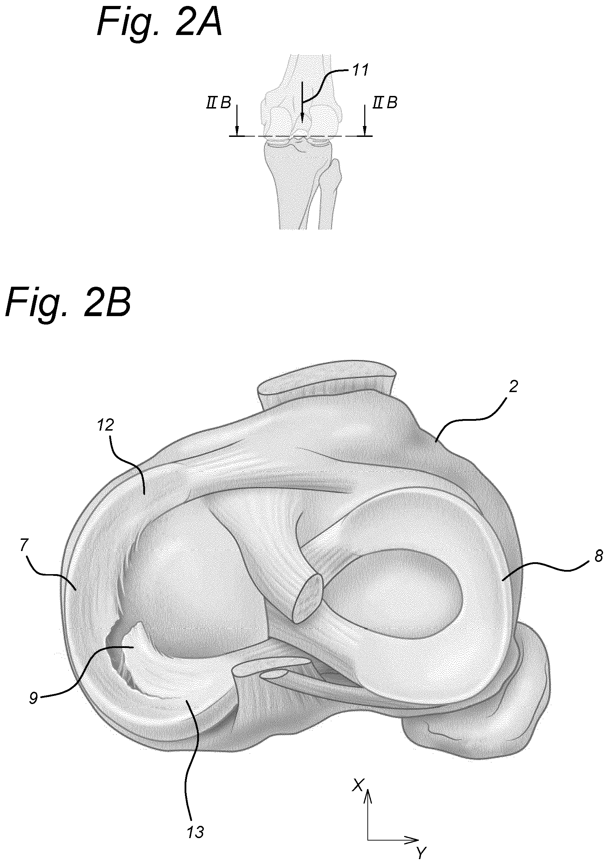

Fig. 2 shows a top-down perspective view of known anatomical structures within the human knee. The viewpoint is indicated by arrow 11. The figure illustrates the tibia 2, medial meniscus 7, and lateral meniscus 8. The figure also shows a flap tear 9 of the medial meniscus 7.

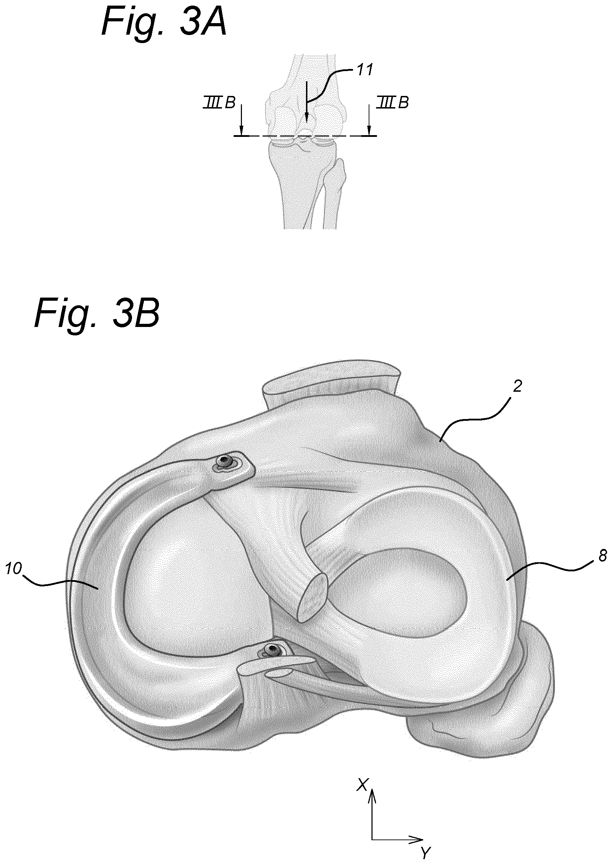

Fig. 3 shows the top-down perspective view of known anatomical structures within a human knee, as in Fig. 2. The figure illustrates the tibia 2 and the lateral meniscus 8. However, the damaged medial meniscus 7 has been removed and replaced by a meniscus prosthesis 10, in particular a Trammpolin® meniscus prosthesis (by Atro Medical B.V., Nijmegen, The Netherlands).

A particular type of meniscus prostheses, such as the Trammpolin® meniscus prostheses, may be available in different sizes. For example, each size may have different anterior, posterior, and mediolateral dimensions. This way, a series of slightly differently sized implants can be manufactured and distributed to hospitals. Alternatively, patient-specific prostheses may be manufactured using techniques such as 3D printing. The techniques disclosed herein may be useful to determine the correct implant size and/or shape, as well as a position of fixation points for the prosthesis, before the surgery.

In a first example, a software program may determine the size and/or location of an implant based on the shape of the patient's tibia. Specifically, the anterior-posterior size and medio-lateral size of the tibia may be determined from a preoperative image and matched with the anterior-posterior size and medio-lateral size of several differently sized implants. Although this provides an estimate of a size of the implant, the inventors found that such analysis may not provide the greatest accuracy.

In the following, several examples of algorithms are described that may ensure an accurate planning of the size of the implants by using, for example, the shape and curvature of the patient's tibia to find the best implant fit.

First, it was found that the curvature of the outside rim of the meniscus prostheses correlates well with the curvature of the tibia plateau, and therefore may be used as a predictor for the size of the meniscus prosthesis.

Second, the native meniscus horns are well-suited indicators of the implant horns. The exact location of the native meniscus horn attachments on the tibia plateau may be located in dimples in the tibia plateau. The dimples with the steepest slope in the tibia may be the exact location of the native meniscus horn attachments.

By using these two pieces of information, either alone or in combination, an accurate location and size of the implant may be calculated.

The accuracy of the algorithm may improve the reliability of the suggested size and/or location of the meniscus prosthesis. The surgeon is thus provided with preoperative information about the size and/or location of the future implant.

The curvature of the outside rim of the tibial plateau, in particular the medial plateau of the upper extremity of the tibia, may be a good indicator of the correct size of the implant. It is noted that throughout this description, the terms "proximal extremity of the tibia" and "upper extremity of the tibia" are meant to be the same and may be used interchangeably. The shape of that plateau itself, including any dimples in that plateau, may provide an additional indicator for a more accurate size prediction.

The techniques disclosed herein are described mainly in relation to implants for joints in a human or animal knee. However, the techniques are not limited thereto. With appropriate adjustments, the techniques may be used for other, similar, types of implants.

In the following, a more detailed example of how to size a meniscus prosthesis will be described. It will be understood that the details of the example serve the purpose of illustrating the possibilities, rather than limiting the invention.

First, a reference model of an object, such as a bone, for example a tibia, is provided. This reference model may be determined beforehand, based on e.g. average information of a population of individuals. This reference model may comprise a shape of a typical tibia. Such a shape may be represented, for example, in form of a surface model, which may be stored, for example, in an STL format. Such an STL (stereolithography) format is known in the art per se.

Second, image data of a patient is obtained, for example by generating an MRI or a CT image dataset of a portion of a patient, where the prosthesis is to be implanted.

The image data may be segmented to detect an object, such as a bone, for example a tibia, in the image data. For example, image segmentation may be applied to the image data to extract a shape model, such as a surface model, of at least part of the object, e.g. of at least part of the tibia. Again, such a surface model may be stored in the STL format, although that is not required. Alternatively, the segmentation may be stored in a volumetric dataset comprising a plurality of voxels.

Next, the reference model may be registered to the segmented image data. For example, the segmented image data may be transformed until the detected object coincides with the reference model. Transformations that may be used for this, may include translation, rotation, and scaling. Optionally, any affine transformation or even any other non-rigid transformation may be applied to register the segmented image data to the reference model.

A coordinate system may be defined, which has for example an origin and axes that are fixed with respect to the reference model. For example, the X axis is an anteroposterior axis (higher values may indicate a more posterior position), the Y axis is a medial axis (higher values may indicate a position closer to the space in between the legs of the patient), and the Z axis is a longitudinal axis (higher values may indicate higher points, assuming a standing patient). Of course, a different coordinate system may be used alternatively. Since the segmented image data is registered with the reference tibia, the coordinate system can be used to identify certain portions of the tibia in the segmented image data.

The points on the surface of the tibia in the segmented image data are determined. In certain implementations, these points are arranged in triangles in the shape model to define a triangularized surface of the tibia.

Additional image processing may be performed to determine the curvature of the anterior portion of the tibia, where the meniscus prosthesis is to be located. To this end, the points of the surface of the tibia, which are within a predetermined region relative to the coordinate system or relative to the reference model, may be selected. This predetermined region corresponds to the region where the tibia plateau is expected to be, for example based on knowledge obtained from a large population of patients. This region may be defined to be large enough, so that the tibia plateau is inside the region for most (if not all) patients. For example, the region is a region including a medial portion of the upper extremity of the tibia. In other words, points above a certain height value (i.e. a Z-coordinate larger than a certain threshold), and closer to the medial side of the tibia (i.e. a Y coordinate larger than a certain threshold).

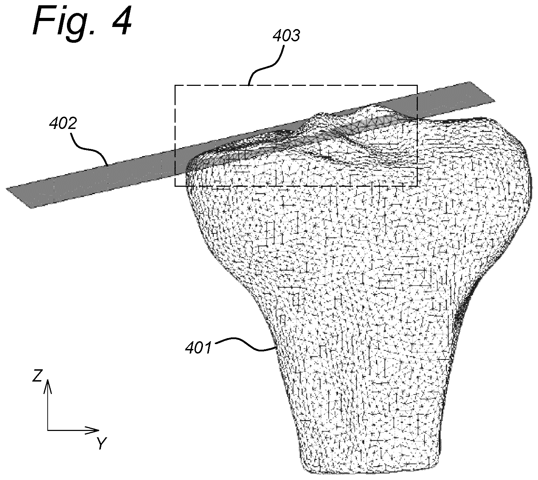

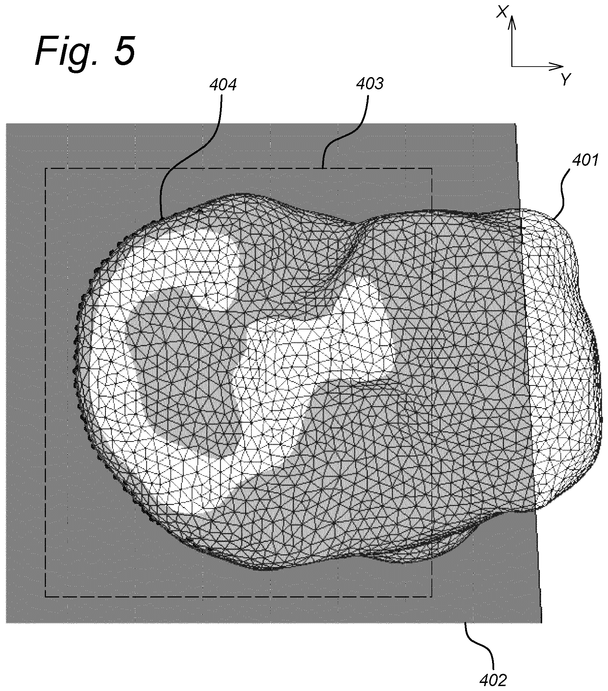

Fig. 4 shows an example, wherein the shape model of the object (tibia) 401 and the plane 402 are illustrated. The reference model is not shown. The medial portion of the upper extremity, where the plane 403 is fitted, is indicated at reference numeral 403. Fig. 5 shows a top view of the same situation.

The curvature of the tibial plateau may be determined based on the points in this region using image analysis techniques. This fitting may be performed using, for example, a least sum of squared errors optimization to minimize the distance of the points to the plane. An initial position of the plane may be determined with respect to the reference model. The plane may be iteratively moved and/or rotated in order to minimize the sum of squared errors. Outliers (points very far away from the initial position of the plane) may be discarded before fitting the plane. For example, the plane may be fitted to the points repeatedly, a number of times, wherein after each fit the points that are furthest away from the plane may be removed, until all remaining points have a distance to the plane that is within a certain tolerance, i.e. below a certain threshold.

Next, the intersection of the surface of the segmented tibia with the plane may be determined. This intersection may constitute a collection of points. For every X-coordinate of a plurality of X-coordinates, a point with the maximal Y-coordinate may be found within this collection of points. That is, for a lot of positions distributed along an anterior-posterior axis, the most lateral intersection of the object with the plane is determined.

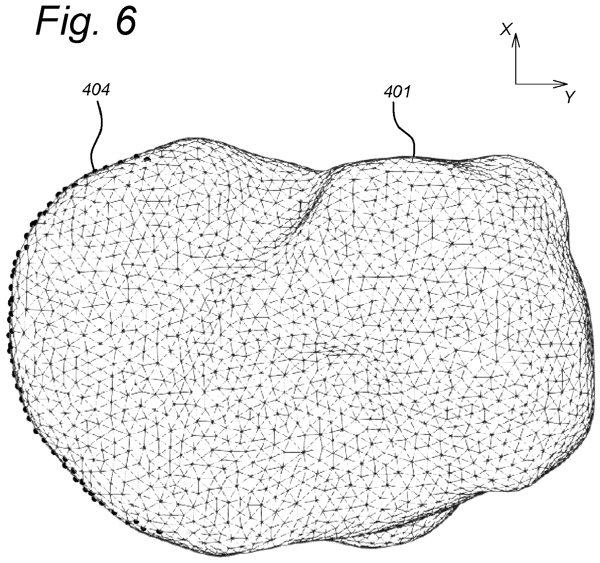

Fig. 5 shows the intersection points 404 thus found, which may be arranged along a curve. Fig. 6 shows the same situation, but without the plane 402. These intersection points provide important information, because they are related to the meniscus. The meniscus prosthesis may be sized based on these points. These points are hereinafter referred to as medial boundary points of the proximal extremity of the tibia, or in a shorthand notation: boundary points. Although a specific algorithm is disclosed herein to detect these boundary points, it will be understood that the skilled person may be able to determine the boundary points using a different algorithm. The present disclosure is not limited to a specific method of determining the boundary points.

A curve may be found by fitting a curve through the boundary points thus found. This fitting may be performed by any suitable known curve fitting technique, such as spline fit. This curve may be the basis for determining a size of a meniscus prosthesis specially adapted to the patient.

Alternatively, a plurality of predetermined curves, associated with a plurality of available differently sized meniscus prostheses, may be rigidly fitted to the boundary points. For this fitting procedure, an ICP (iterative closest point) technique may be employed, based on the distance between each of the boundary points and the predetermined curve associated with a particular meniscus prosthesis. The error that remains after fitting may be used as an indicator of the suitability of that meniscus prosthesis for this particular patient. This error may be calculated, for example, based on the sum of squared errors, wherein the errors are the distance from each boundary point to the curve. This error may be normalized according to the number of boundary points. Alternatively, the boundary points may be resampled, or the number of boundary points may be reduced, to obtain a fixed number of boundary points to be fitted. In such a case, this normalization may be omitted. Also, the different errors obtained for the different curves for a given set of boundary points may be compared to each other even without any normalization taking place.

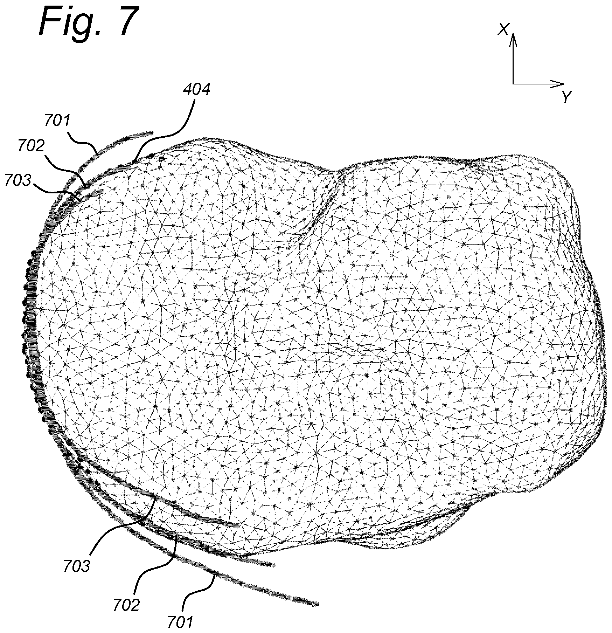

Fig. 7 shows a plurality of different curves 701, 702, 703, associated with different implant sizes, which curves are rigidly fitted (i.e. by applying rigid transformations to the curves, such as translation and/or rotation transformations) to the boundary points 404. In this case, the error that remains after fitting is the smallest for the curve 702. That is, the error that remains after fitting of either of the curves 701 and 703 is larger than that of curve 702. This provides an indication that the implant associated with curve 702 is to be selected for this patient. Therefore, in certain implementations of the techniques disclosed herein, the system outputs an indication of the implant size associated with the best fitting curve.

Moreover, each implant may also have a predetermined target placement location relative to the curve associated with it. Specifically, the curve may coincide with the outer boundary of the prosthesis, especially in case of a meniscus prosthesis. Other relative placement locations are also conceivable. Therefore, the system can show a position of the implant corresponding to the fitted curve. To this end, the fitting of the curve may have some restrictions. The restrictions may include some limitations on rotations and positions. For example, the implant cannot be placed too far outside of the plateau, and the horns should be in line with the AP direction.

The system may comprise a database with information associated with a plurality of different implant sizes. For example, information may comprise: the curve related to the boundary points of the tibia; the predicted spatial location of the implant within the patient body with respect to the curve; locations of fixation elements (e.g. screws, screwholes) of the implant. More generally, the information in the database may comprise: a curve to be fitted to boundary points of an object to which the implant is to be fixed, the spatial location of the implant after implantation; and locations of fixation elements (e.g. screws, screwholes) of the implant.

In certain embodiments, other features may be extracted to determine the size and/or location of the implant. These other features may be extracted from the image data in addition to the boundary points, or instead of the boundary points. These other features may also be compared to corresponding features stored in the database for the implant sizes. Alternatively, the other features may be used to determine the size of a patient-specific implant to be made by means of, for example, three-dimensional printing.

For example, a dimple in the surface of the object may be found by analyzing the shape model. In general, a dimple may be a recess in the surface of the three-dimensional shape model. The surface of the tibia typically has dimples at the locations where implant may be attached. These dimples may be detected in the shape model. In particular the location of the posterior horn 12 (Fig. 2) of the meniscus provides important information about the implant size. The location of the anterior horn 13 may also provide further information. The combined information of the detected features (e.g. boundary points, horn locations) may be used to determine the best fitting implant size.

One way of detecting these dimples is based on searching points on the surface, which points satisfy the condition that a normal of the surface satisfies certain conditions. In certain embodiments, the normal may be determined by determining the normal of a triangle of a triangular mesh representing the surface of the tibia.

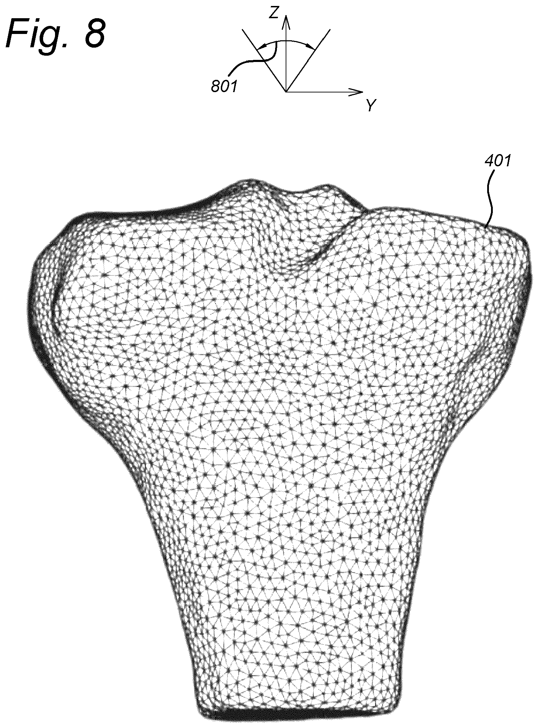

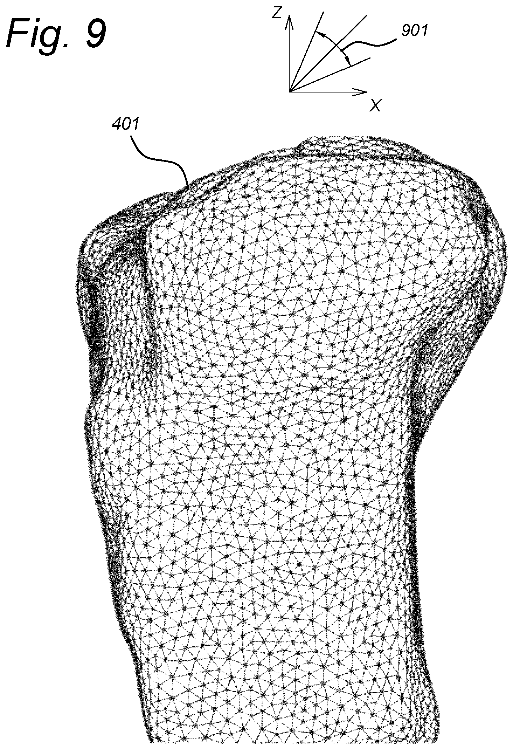

Fig. 8 and 9 illustrate an angular range that may be used to select points on the surface that correspond to horn attachment points. As shown in Fig. 8, the angle of the normal of the surface of the tibia with respect to the vertical axis in the YZ plane is to be within a first range, illustrated at 801, for example in the range of -35 degrees to +35 degrees. However, the exact boundaries of the range are not a limitation. As shown in Fig. 9, the angle with a diagonal in the XZ plane should be within a second range, illustrated at 901, for example in the range of -22 degrees to +22 degrees. In other words, since the diagonal is at 45 degrees, the angle with the X-axis in the XZ plane should be in a range of 23 degrees to 67 degrees. However, the exact boundaries of the range are not a limitation. It will be understood that the above angular range used to detect horn attachment points are provided by means of example. In general, a dimple or recess in the surface of the tibia plateau may be the horn attachment point. In case more dimples or recesses occur in the tibia plateau, in particular a relatively steep or deep dimple or recess may be the horn attachment site.

In certain embodiments, among the points that satisfy the conditions on the normal, only points within a certain distance, for example 1 mm, from the fitted plane are selected as a possible location of the posterior horn.

In certain embodiments, among any remaining candidates of locations of the posterior horn, one is selected as final estimate of posterior horn. For example, the most posterior candidate is selected as final estimate of the posterior horn location.

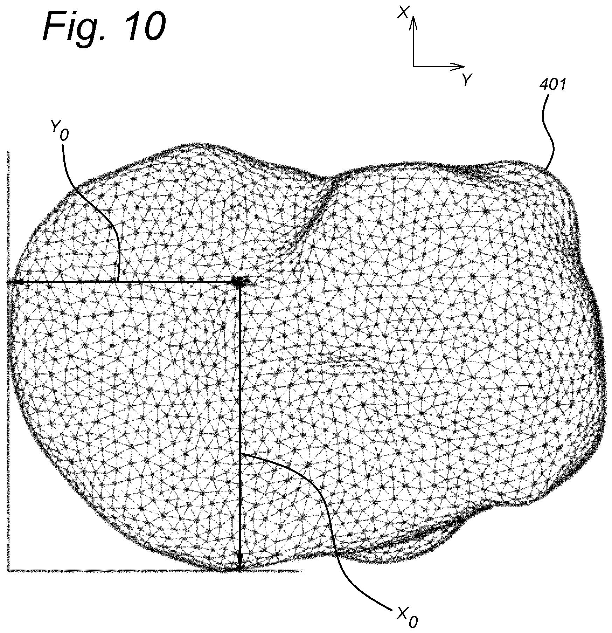

As shown in Fig. 10, when the posterior horn location has been finally estimated, the distance x₀ along the anteroposterior (X) axis from the posterior horn location to the boundary point of the tibia in the fitted plane may be calculated. Also, the distance y₀ along the mediolateral (Y) axis from the posterior horn location to the boundary point of the tibia in the fitted plane may be calculated. These two distances provide additional features that are useful to determine the size of a meniscus prosthesis. These two distances may be compared with predetermined, corresponding distances on the implant. Preferably an implant is selected in which these values match (up to a certain error level).

The database of implant information may comprise information about the posterior horn location in relation to the location of a posterior screw hole of the implant. For example, depending on the design of the implant, the posterior screw hole of the implant may be located on a portion of the implant that is intended to cover the posterior horn. The database may further comprise information about the location of any particular recognizable feature in the shape model in relation to a specific portion of the implant. This information may be used to detect any mismatch between the location in the shape model and the corresponding location in the implant. Thus, the implant size with the least mismatch may be chosen.

As indicated in Fig. 10, the location of the posterior horn, once detected, may be measured relative to the boundary points of the tibial plateau. For example, the distance x0 from the posterior horn to the boundary in anteroposterior (X) direction and the distance y0 from the posterior horn to the boundary in medial (Y) direction may be measured. These values x0 and y0 may be compared with corresponding values of the differently sized implants.

For example, the size determination of the implant may be performed in two steps: first, a plurality of implant sizes may be selected that have an error in respect of the curve fit, below a threshold value. Second, among the plurality of implant sizes selected in the first step, the mismatch between the detected location of the posterior horn and the posterior screw hole may be determined. In the second step, the implant size with the smallest mismatch may be selected. Optionally, also a mismatch between the detected anterior horn and the anterior screw hole may be taken into account.

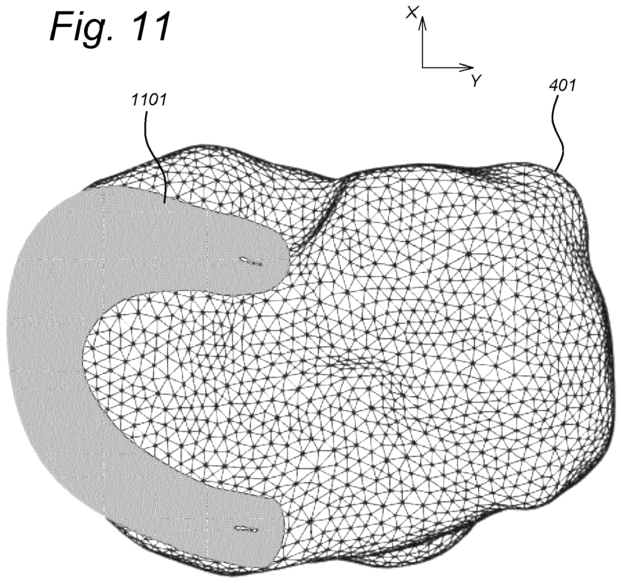

Fig. 11 shows the tibia 401 with the location of the implant 1101, as determined by the above algorithm.

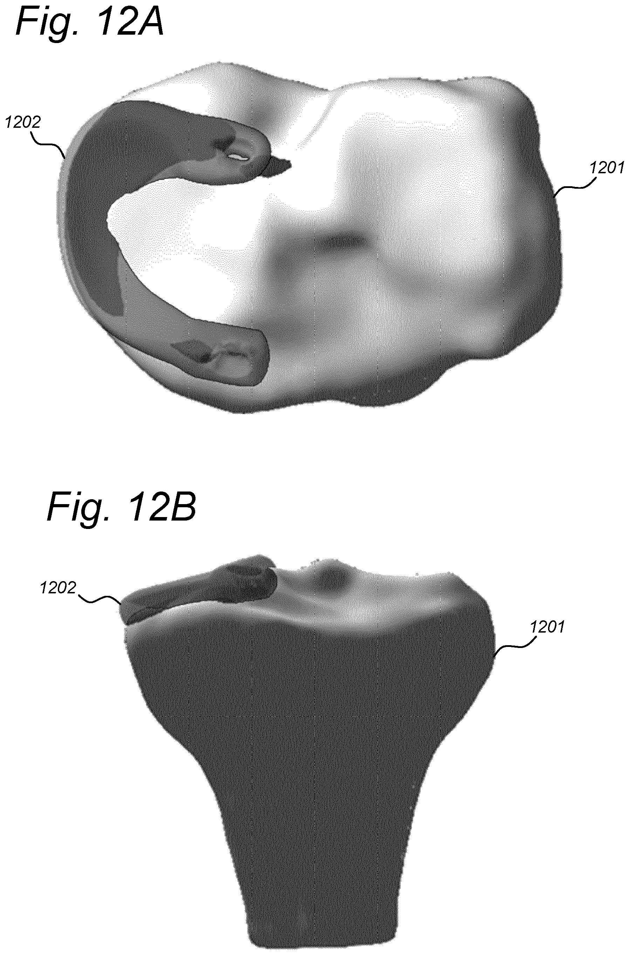

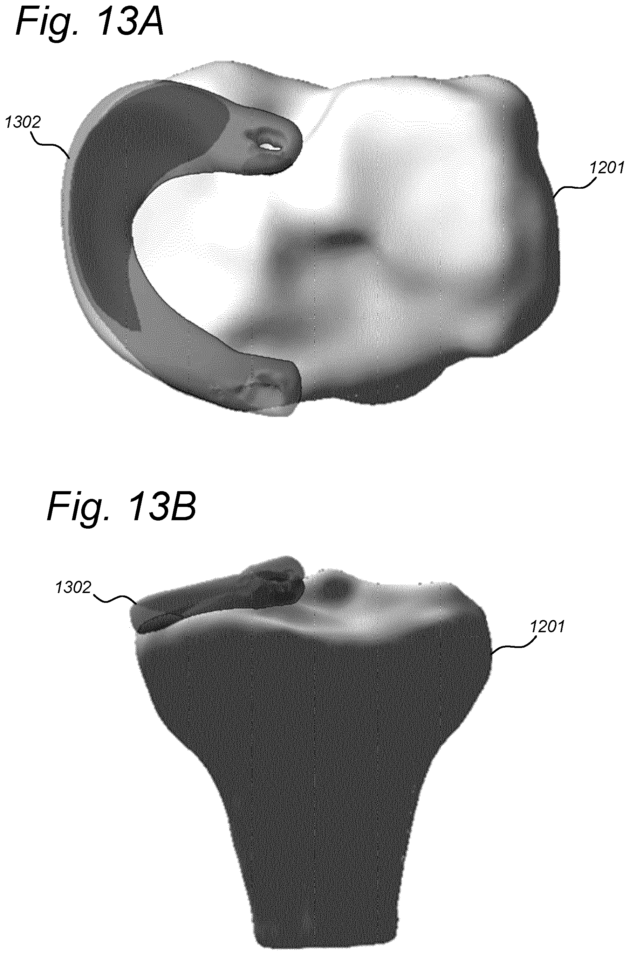

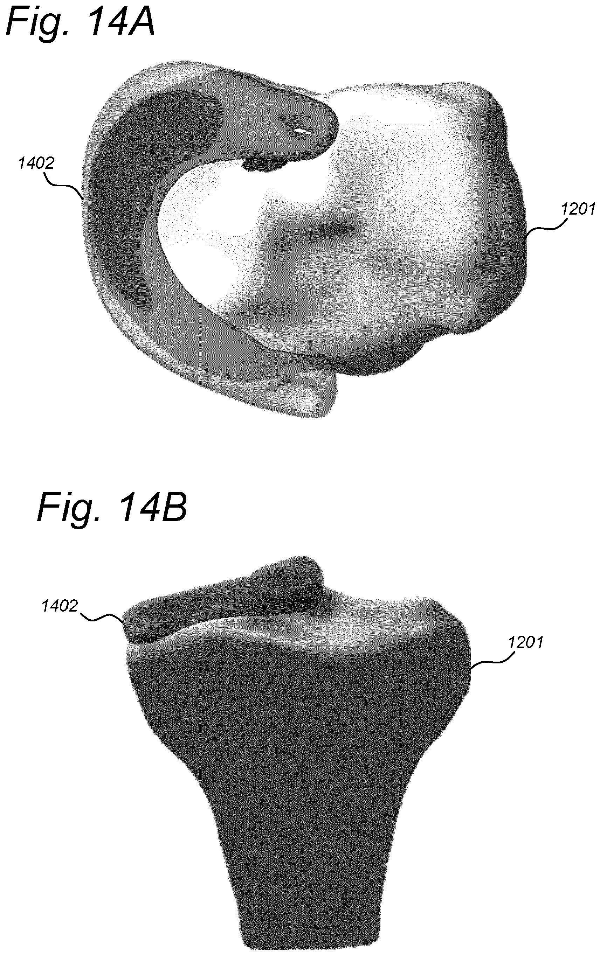

For purpose of illustration, Fig. 13 shows two views of a tibia 1201 with an implant 1302, wherein the implant size was determined by the above described algorithm. Fig. 12 shows two views of the tibia 1201, with a differently sized implant 1202, not determined by the above described algorithm. The implant 1202 appears to be too small. Fig. 14 shows two views of the tibia 1201, with another implant 1402, wherein the implant size was not determined by the above described algorithm. The implant 1402 appears to be too large. Fig. 13 shows that the above algorithm results in a properly suited size of implant 1302.

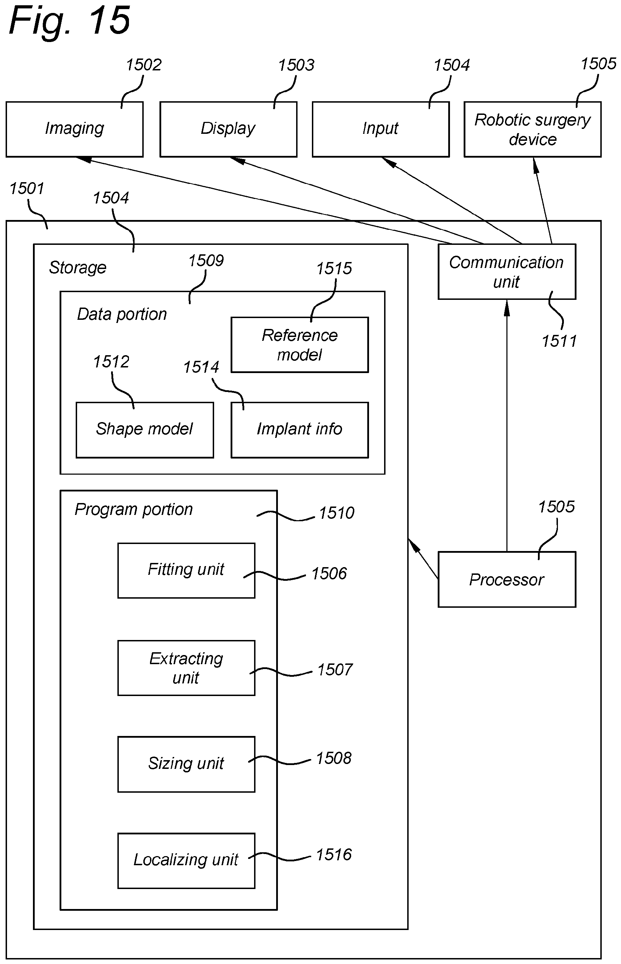

Fig. 15 shows a block diagram of an example implementation of a system 1501 for sizing an implant. For example, the system 1501 comprises an electronic device including a processor 1505, such as a central processing unit, a communication unit 1511, which may support USB connection, wired and/or wireless network connections, and other kind of interface to external devices, such as a medical imaging device or image server 1502, display 1503, input device (mouse, keyboard, touch screen) 1504. In certain embodiments, it is even possible to connect the system 1501 to a robotic surgery device 1505, that uses the implant size and/or position transmitted from the system 1501 to the robotic surgery device 1505, to control a robot to automatically grab and/or position the implant. The processor 1505 may control the communication unit 1511 to receive a shape model of the object from the imaging device 1502, and may store the received shape model in the data portion 1509 of the storage 1504. Storage 1504 may comprise transient and/or non-transient data storage facilities, such as FLASH memory, read-only memory, random access memory, and magnetic storage media. The storage 1504 may be divided in a data portion 1509 and an instruction portion 1510. The data portion 1509 may comprise shape models 1512 and a database of information about several implants 1514, in particular about different implant sizes of a series of available implants, and a reference model 1515. The instruction portion 1510 may comprise instructions of one or more computer programs, to implement certain functionality of the system by causing the processor 1505 to perform certain control actions and/or computations. These instructions may be divided into units.

It will be understood that the system 1501 may be implemented as a standalone device, such as a computer. Alternatively, the system may be implemented as a client-server system or cloud-based system, in which certain data and/or instructions may be stored and/or executed on a different server, remote from the display 1503 and input device 1504. In that case, a client device may be provided at the display 1503 and input device 1504 to control communications with the remote server. It will be understood that the display 1503 and the input device 1504 are optional devices. The system 1501 may be alternatively controlled by for example the imaging system 1502 or another external device.

The system may be configured to store a dataset, comprising a three-dimensional shape model 1512 of an object, in the storage means 1504. The system may, for example, be configured to receive a segmented shape model 1512 via the communication unit 1511, or it may be configured to segment a three-dimensional imaging dataset, such as a CT dataset or MRI dataset, to obtain the shape model of the object. Such segmentation techniques are not described herein in greater detail.

The system may further be configured to store a reference model 1515 in the storage means 1504. The reference model may be, for example, be designed manually when developing the system, based on knowledge of the anatomy of the part of the body that the implant is destined for. For example, the reference model may be a reference shape model of a bone to which the implant is to be attached.

The system may further be configured to store predetermined information 1514 about a plurality of implants or implant sizes. This implant information 1514 may be stored in form of a database or a list of items, for example. The implant information 1514 may, for each of a plurality of implant sizes, contain associated information such as a matching shape of the bone to which the implant is to be attached or fixed, fixation points, and the like. This implant information may be, for example, determined manually when developing the system, based on knowledge of the anatomy of the part of the body that the implant is destined for.

The system may further comprise a fitting unit 1506 for fitting a reference model 1515 to the shape model 1512 of the object, and defining a coordinate system relative to the fitted reference model. The coordinate system provides a reference for the orientation and important regions of the shape model. For example, the fitting unit 1506 may be configured to perform the fitting by applying successively different transformations to the reference model, until the reference model corresponds best to the shape model. The fitting unit 1506 may be implemented using standard medical image registration techniques.

The system may further comprise an extracting unit 1507 for extracting shape related features from the shape model, based on the coordinate system. For example, certain portions of the object may be identified and/or measured, based on the shape model.

The system may further comprise a sizing unit 1508 for determining a size of an implant, based on the extracted shape related features. To this end, the extracted shape related features may be compared to the implant information 1514 for each of a plurality of candidate implant sizes. The implant size with the best matching implant information may be selected as the most suitable implant size.

Alternatively, the sizing unit 1508 may generate one or more parameter values indicative of the shape and size of the ideal implant (for example a scaling factor), so that the ideal implant may be manufactured by means of e.g. 3D printing, by applying the parameter values to a parameterized model of the implant.

The determined size of implant may be used by a surgeon, for example, to select a properly sized implant and implant it into the patient depicted by the shape model.

The shape model 1512 may represent an object within a patient body, for example a bone of a patient, in particular a part of a bone around a joint, for example a tibia or femur or a portion of a tibia and/or femur around the knee joint. It is also possible that the extracting unit 1507 can extract information from shape models of several objects in the body that are close to the designated place of the implant. For example, features of both tibia and femur may be detected and taken into account when determining the implant shape and size.

The extracting unit 1507 may identify a region relative to the reference model, using the coordinate system, and searching for the features of the shape model in the identified region. For example, the reference model may comprise a portion that corresponds to a region where the implant would be fitted. After the reference model is fitted to the shape model, this region may thus be identified in the shape model using the coordinate system. The relevant region may then be processed to find relevant features such as a rim or a dimple.

The extracting unit 1507 may be configured to extract information indicative of a curvature of an outside rim of the object, for example a curvature of an outside rim of a proximal (or upper) extremity of a tibia. For example, in case the implant comprises a medial meniscus prosthesis, the curvature of a medial rim of the proximal extremity of the tibia may be extracted. On the other hand, in case the implant comprises a lateral meniscus prosthesis, the curvature of a lateral rim of the proximal extremity of the tibia may be extracted.

For example, the extracting unit 1507 may be configured for selecting a subset of the dataset corresponding to at least a portion of the shape model within a predetermined region, defined with respect to the coordinate system, where the implant is to be placed, fitting a plane to the shape model based on the subset of the dataset, determining intersection points of the shape model with the fitted plane, and optionally fitting a curve to the intersection points of the three-dimensional shape model with the fitted plane.

The sizing unit 1508 may be configured for selecting the size of the implant based on the intersection points or fitted curve. For example, the sizing unit 1507 may be configured for comparing the curvature of the outside rim of the object, which may be represented by the intersection points or the fitted curve, to a corresponding curve associated with the implant. For example, the corresponding curve associated with the implant may be an outer rim of the implant. For example, an error function for a particular implant size may be defined representing a mismatch between the outside rim of the object and the corresponding curve associated with the implant. This error function may be calculated based on the distance between certain points on the outside rim and their corresponding points on the curve associated with the implant. The minimal error function for a particular implant size may be determined by applying rigid transformations (e.g. translation, rotation) to the rim and/or curve, until the smallest possible error function for a particular implant size has been determined. This procedure may be repeated for the different available implant sizes. The implant size having the smallest error function may be selected as the best implant size for a particular patient.

The extracting unit 1507 may be configured for determining a surface of the shape model in at least a specific region relative to the coordinate system. For example, the shape model comprises a surface mesh, of which a certain portion is within the specific region. this portion of the surface mesh within the region may be subject of further analysis. The region may correspond to a specific region of the reference model. For example, the region may correspond to a region of the reference model where a certain anatomical part is. For example, the region may correspond to a portion of a bone close to a joint. In case of a meniscus prosthesis, for example, the region may correspond to a medial plateau of the upper extremity of the tibia. In case of a lateral meniscus prosthesis, the region may correspond to a lateral plateau of the upper extremity of the tibia. In certain implementations, the region may be a slice with a certain thickness around the fitted plane, i.e. all points with a distance from the fitted plane that is smaller than a certain predetermined threshold, such as 1 millimeter or 2 millimeters.

The extracting unit may be configured to extract a certain feature of the surface, for example a dimple, that corresponds to a common anatomical shape. A dimple may be detected, for example, by mathematical analysis of the normal of the surface. For example, a derivative analysis may be performed to detect a dimple e.g. as a local minimum. Such mathematical methods are known in the art per se.

Once a dimple has been detected, several features of the detected dimple may be used to determine a properly sized implant. For example the width and/or the depth of the dimple may be used as features. Alternatively, the location of the dimple on the surface of the object may be used as a feature to determine the size of the implant. The location may be determined relative to the coordinate system of the registered reference model, for example. Alternatively, the distance of the dimple to certain well-recognizable points may be calculated.

For example, the distance from the dimple to the medial rim of the upper extremity of the tibia may be calculated and used as a feature that is relevant for the implant size. For example, the distance may be measured in two orthogonal directions; for example, the distance of the dimple to the rim in anteroposterior direction and the distance to the rim in mediolateral direction. The rim itself may be estimated using the medial boundary points of the upper extremity of the tibia, as described above.

The features about the dimple (e.g. dimple shape or size or location) may be used by the sizing unit to determine a suitable implant size. The features may be compared with reference values provided in the database for each implant size. The different features may be weighted differently, to take the importance of each feature into account. In the same way, the curvature may be taken into account with a certain weight.

The sizing unit 1508 may be configured for associating the location of the detected dimple with a location of an attachment element of the implant for attaching the implant to the object. For example, an implant may be designed with e.g. a screw hole or a hole for a pin, or another connection element to fix the implant to the object, at a place where the object is expected to have a dimple. In such a case, differently sized implants may have their screw holes at different locations and optionally with a different distance between a pair of screw holes. Also, the size of the implant may have a relation with the location of the dimple/screw hole, such as the distance from the dimple to the rim.

In certain implementations, if the dimple is not detected inside a specific region where the dimple is expected, but outside such a region, then the sizing unit may ignore the dimple. This way, erroneous sizing may be avoided.

In certain implementations, different features may be combined to determine the size of the implant with greater reliability. For example, for each feature the sizing unit may determine a numeric value that expresses a mismatch between a detected feature and the corresponding feature of a particularly sized implant. Such a mismatch may be referred to as an error value. Each type of feature may be assigned a certain weight, so that more important features have more influence on the final size. The determined error value of each feature may be multiplied by its weight. The weighted error values may be summed together to obtain a weighted sum of the error values for a particular implant size, which may represent an overall mismatch value of the implant in respect of the object represented by the three-dimensional shape model. The overall mismatch value may be determined for a plurality of candidate implant sizes. Finally, the implant size having the smallest overall mismatch value may be determined as the most suitable implant size in view of the detected features.

The system may further comprise an optional localizing unit 1516. The localizing unit 1516 may determine an optimal location of the implant inside the body. For example, the optimal location may be determined relative to the three-dimensional shape model, based on the determined features. The location may be determined by mapping the determined features in the shape model to corresponding reference points on the implant. A desirable distance between such a detected feature and a reference point on the implant may be determined beforehand and stored in a database, for example. In the example of a meniscus prosthesis, the boundary points may be mapped to certain boundary points of the prosthesis, wherein the desired distance or vector between the boundary points of the proximal extremity of the tibia and the corresponding points on the prosthesis may be pre-stored in the data portion 1509 of the storage means 1504. Additionally or alternatively, the dimples in the shape model representing the horns may be mapped to corresponding attachment points on the meniscus prosthesis, for example the location of screw holes.

The processor 1505 may be configured to output the determined location and/or the determined size via the communication unit 1511, for example to display the information on the display 1503 and/or to send the determined location and/or the determined size to robotic surgery device 1505.

After the correct implant size has been determined by the sizing unit 1508, the implant may be provided by manufacturing it or by taking it from an inventory of differently sized implants. After that, the implant may be implanted into the patient.

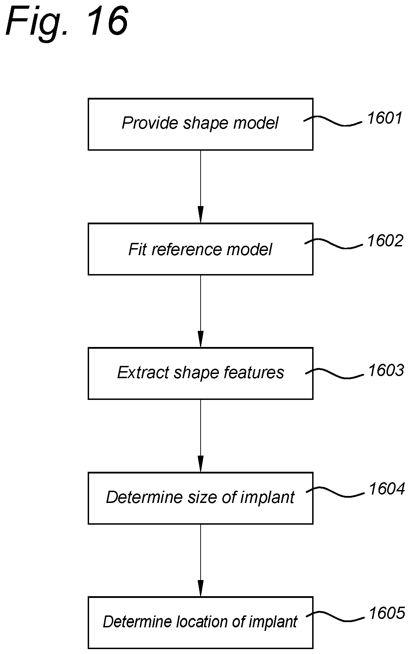

Fig. 16 illustrates a method of determining a size of an implant. The method may be performed, for example, by a processor or a computer system. The method may be embodied by a non-transitory computer readable media comprising instructions that, when executed by a processor or computer, cause the processor or computer to perform the method. The method may start at step 1601 by providing a dataset comprising a three-dimensional shape model of an object. The three-dimensional shape model of the object may be generated, for example, by scanning a patient in an image scanner, such as a computed tomography device or a magnetic resonance imaging device. The resulting three-dimensional image dataset may be segmented using state of the art segmentation techniques, to segment an object represented by the image dataset. This segmented object may represent a shape model of the object. The segmented object may further be transformed by modeling the outside surface of the object, for example, as a point cloud or a triangular mesh as alternative representations of a shape model.

In step 1602, a reference model is fit to the shape model of the object. That is, a reference model may be provided beforehand based on knowledge of general anatomy of the population for which the implant is intended. The reference model represents a similar, non-patient-specific object. The patient-specific object as represented by the shape model may be fitted to the reference model, or vice versa.

For ease of the further processing steps, a coordinate system may be defined relative to the fitted reference model. That is, the reference model may have a fixed origin and coordinate axes associated therewith, and after the reference model has been fitted to the shape model, the coordinate system of the reference model may be applied to the shape model, so that specific regions of anatomical relevance may be easily identifiable based on the coordinate system.

Based on the coordinate system, in step 1603 certain features of the shape model at or around certain expected locations of the object may be assessed, using any feature extraction method as known in the art or as described herein.

In step 1604, a size of the implant may be determined, based on the extracted shape related features. For example, the features extracted from the shape model may be compared with certain predetermined reference values of each of a plurality of candidate implant sizes. Based on the comparison, the best matching candidate implant size may be selected.

After the size of the implant has been established, the implant of that size may be provided for implantation.

In optional step 1605, an optimal location of the implant may be determined based on the determined features. The location may be determined relative to the three-dimensional shape model. The location may be determined by mapping the determined features in the shape model to corresponding reference points on the implant. A desirable distance between such a detected feature and a reference point on the implant may be determined beforehand and stored in a database, for example.

It will be understood that the techniques have been disclosed in detail hereinabove by using the medial meniscus prosthesis as an example. However, similar techniques may be applied to determining the size and/or location of other kinds of implants, such as, for example, lateral meniscus prosthesis.

The techniques disclosed herein may also be applicable, for example, for replacing the labrum in the shoulder. The labrum is a meniscus-like rim on the glenoid which is attached to the capsule of the shoulder joint. The labrum plays an important role in the stability of the joint and increases the contact area of the glenohumeral joint. An implant, which may for example be made of polymer, may be able to replace the anterior labrum of the shoulder to raise the rim and increase the surface area of the anterior glenoid. The anterior curvature of the glenoid resembles the medial curvature of the tibia plateau. The techniques disclosed herein may be used, for example, to determine an optimal size of a labrum prosthesis. For example, the reference model 1515 may represent at least a portion of a shoulder joint, the fitting unit 1506 may be configured to fit the reference model to a three-dimensional dataset comprising a shape model 1512 representing a shoulder area of a patient. The extracting unit 1507 may be configured to extract features of the shape model 1512 relating to the shoulder joint and labrum. For example, for an anterior labrum prosthesis, the anterior curvature of the glenoid may be detected by the extracting unit 1507. The implant information 1514 may comprise, for each candidate implant size, a corresponding optimal anterior curvature of the glenoid. The sizing unit 1508 may be configured to compare the extracted anterior curvature of the glenoid with the implant information 1514 of a plurality of the candidate implant sizes, to determine an optimal implant size. The implant information 1514 may comprise a mapping of specific points on the anterior glenoid to specific corresponding points on the implant. The localizing unit 1516 may determine an optimal position of the implant based on the extracted features and the mapping.

As another example, the techniques may be used to determine the size of an implant for total knee replacement, or other knee related implants. The boundary points of the tibial plateau may be compared to a corresponding curve of a total knee replacing implant. Similarly, the detected dimple may be compared to a corresponding point of the artificial tibia of a total knee replacing implant. Other implant types of which the size and/or location may be determined using the techniques disclosed herein include hip prostheses and shoulder prostheses.

Some or all aspects of the invention may be suitable for being implemented in form of software, in particular a computer program product. The computer program product may comprise a computer program stored on a non-transitory computer-readable media. Also, the computer program may be represented by a signal, such as an optic signal or an electro-magnetic signal, carried by a transmission medium such as an optic fiber cable or the air. The computer program may partly or entirely have the form of source code, object code, or pseudo code, suitable for being executed by a computer system. For example, the code may be executable by one or more processors.

The examples and embodiments described herein serve to illustrate rather than limit the invention. The person skilled in the art will be able to design alternative embodiments without departing from the spirit and scope of the present disclosure, as defined by the appended claims and their equivalents. Reference signs placed in parentheses in the claims shall not be interpreted to limit the scope of the claims. Items described as separate entities in the claims or the description may be implemented as a single hardware or software item combining the features of the items described.

## Claims

1. A system for determining a size of an implant, comprising
a storage means (1509) configured to receive and store a dataset comprising a three-dimensional shape model of an object;
a fitting unit (1506) configured to fit a reference model to the shape model of the object, and define a coordinate system relative to the fitted reference model;
an extracting unit (1507) configured to extract shape related features from the shape model, in dependence on the coordinate system;
a sizing unit (1508) configured to determine a size of the implant, based on the extracted shape related features.

2. The system of claim 1, wherein the sizing unit (1508) is configured to:
compare the extracted shape related features to corresponding features associated with a plurality of differently sized implants; and
select a particularly sized implant based on a result of the comparing.

3. The system of any preceding claim, wherein the implant is to be fitted to the object, and wherein the object comprises a bone or at least part of a tibia, and/or the implant is a meniscus prosthesis.

4. The system of any preceding claim, wherein the extracting unit (1507) is configured to identify a region relative to the reference model, using the coordinate system, and search for the features of the shape model in the identified region.

5. The system of any preceding claim, wherein the shape related features include information indicative of a curvature of an outside rim of the object.

6. The system of claim 5, wherein the object comprises at least part of a tibia, and wherein the information indicative of a curvature of an outside rim of the object comprises information indicative of:
a medial boundary of a proximal extremity of the tibia if the implant is a medial meniscus prosthesis, or
a lateral boundary of the proximal extremity of the tibia if the implant is a lateral meniscus prosthesis.

7. The system of any preceding claim,
wherein the extracting unit (1507) is configured to:
select a subset of the dataset corresponding to at least a portion of the shape model within a predetermined region where the implant is to be placed;
fit a plane to the shape model based on the subset of the dataset; and
determine intersection points of the shape model with the fitted plane,
wherein the sizing unit (1508) is configured to select the size of the implant based on the determined intersection points.

8. The system of claim 5, 6, or 7, wherein the sizing unit (1508) is configured to compare the information indicative of the curvature of the outside rim of the object or the determined intersection points to a corresponding curve associated with the implant.

9. The system of any preceding claim,
wherein the extracting unit (1507) is configured to:
determine a surface of the shape model in a specific region relative to the coordinate system; and
detect a dimple in the surface,
wherein the sizing unit (1508) is configured to:
determine the size of the implant based on the detected dimple.

10. The system of claim 9,
wherein the sizing unit (1508) is configured to determine the size of the implant based on a location of the detected dimple.

11. The system of claim 9 or 10,
wherein the specific region comprises a medial plateau of an upper extremity of a tibia.

12. The system of claim 9 in dependence on claim 7,
wherein the specific region corresponds to a region within a predetermined distance from the fitted plane.

13. The system of any one of claims 9 to 12, wherein the sizing unit (1508) is configured to associate the location of the detected dimple with a location of an attachment element of the implant for attaching the implant to the object.

14. The system of any preceding claim, further comprising a localizing unit (1516) configured to determine an implant position, where the implant can be placed, relative to the three-dimensional shape model, based on the extracted shape related features.

15. A method of determining a size of an implant, comprising
providing (1601) a dataset comprising a three-dimensional shape model of an object;
fitting (1602) a reference model to the shape model of the object, and defining a coordinate system relative to the fitted reference model;
extracting (1603) shape related features from the shape model, in dependence on the coordinate system;
determining (1604) a size of the implant, based on the extracted shape related features.
